**Europäisches Patentamt**

**European Patent Office**

**Office européen des brevets**

(11) Numéro de publication: **0 214 962**
**B1**

(12) **FASCICULE DE BREVET EUROPEEN**

(45) Date de publication du fascicule du brevet: **06.06.90**

(21) Numéro de dépôt: **85901464.9**

(22) Date de dépôt: **29.03.85**

(86) Numéro de dépôt international:
**PCT/FR85/00064**

(87) Numéro de publication internationale:
**WO 85/04321 10.10.85 Gazette 85/22**

(51) Int. Cl.⁵: **A 61 C 8/00**

(54) **IMPLANT DENTAIRE POUR LA FIXATION DE PROTHESES DENTAIRES FIXES.**

(30) Priorité: **29.03.84 FR 8405129**

(43) Date de publication de la demande:
**25.03.87 Bulletin 87/13**

(45) Mention de la délivrance du brevet:
**06.06.90 Bulletin 90/23**

(84) Etats contractants désignés:
**AT BE CH DE GB IT LI LU NL SE**

(56) Documents cités:
**FR-A-1 551 020**
**FR-A-2 040 781**
**FR-A-2 050 198**
**FR-A-2 302 715**
**US-A-3 474 537**

(73) Titulaire: **SCORTECCI, Gerard**
**10, rue du Soleil**
**F-06000 Nice (FR)**

(72) Inventeur: **SCORTECCI, Gerard**
**10, rue du Soleil**
**F-06000 Nice (FR)**

(74) Mandataire: **Hautier, Jean-Louis**
**Cabinet Hautier Office Méditerranéen de Brevets**
**d'Invention et de Marques 24 rue Masséna**
**F-06000 Nice (FR)**

Courier Press, Leamington Spa, England.

**Description**

L'invention a pour objet un implant dentaire pour la fixation de prothèses dentaires fixes.

L'implant est l'infrastructure généralement métallique destinée à soutenir une prothèse dentaire. Il permet de remplacer les piliers naturels que sont les dents, par des piliers mécaniques, placés soit dans l'os mandibulaire, soit dans l'os maxillaire.

L'essentiel dans la pose d'un implant dentaire est d'obtenir un blocage immédiat dans l'os. Une mobilité même faible de l'implant dans l'os entraîne à terme un rejet. Une fois l'implant mis en place dans l'os, un bague taraudée ou vis est mis en place sur la partie extérieure qui est en général une tige filetée. Sur ladite bague taraudée ou vis est fixée, par un ciment, la prothèse dentaire.

L'état de la technique peut être défini par le brevet de Monsieur Jean-Marc JUILLET déposé le 12 juin 1972 sous le No. 72 21113 et publié le 2 janvier 1974 sous le No. 2.188.445.

Ce brevet décrit un implant anatomique endo-osseux et son procédé d'insertion. L'invention de Monsieur Jean-Marc JUILLET concerne un implant anatomique endo-osseux comprenant une grille surmontée d'une ou de plusieurs tiges dites "faux-moignon" pour la fixation de prothèses, implant caractérisé en ce que le polygone de sustenation de la grille s'étend essentiellement en plan horizontal, cette grille étant conformée pour être disposé dans l'os mandibulaire ou maxillaire, suivant un ou plusieurs plans issus ou voisins d'un plan horizontal de manière telle que la grille pénètre dans une tranchée à un ou plusieurs plans sensiblement horizontaux pratiqués sur la face vestibulaire, linguale ou palatine du corps de maxillaire considéré, tandis que le ou les faux-moignons pénètrent en même temps dans autant de passage verticaux recoupant le ou les plans de la tranchée sensiblement horizontale.

L'invention concerne également un procédé d'insertion d'un tel implant consistant à pratiquer dans un os une découpe correspondant au profil projeté latéralement au dit implant et à insérer latéralement ledit implant dans ladite découpe de préférence par la face vestibulaire de telle sorte que seule dépasse de la crête de l'os l'extrémité du faux-moignon opposée au corps de l'implant.

D'autres implants connus sous le nom de lames de LINKOW peuvent également définir l'état de la technique. Ces lames sont caractérisées par une partie d'accrochage en forme de lame qui est formée de plusieurs jambes d'accrochage qui sont arquées et qui se terminent par des pieds. L'ensemble de cette partie tend à permettre au tissu osseux de venir peu à peu s'imbriquer entre les jambes et les pieds pour ancrer solidement l'implant.

Le mode de réalisation de ces implants et leur mode de mise en place présentent de nombreux inconvénients. Le forage de l'os, en plan vertical sur un diamètre parfois important, est nécessairement profond pour masquer la totalité de la hauteur des implants. Or, bien souvent, l'os ne présente pas une hauteur suffisante et ces implants ne peuvent contourner les obstacles tels que sinus, fosses nasales, nerfs, le forage devant se faire presque toujours verticalement.

Ces types d'implants connus ne peuvent en conséquence s'adapter aux différentes conformations osseuses. Ces implants, essentiellement insérés en plan vetical, présentent en outre de mauvaises caractéristiques de transmission des efforts auxquels ils sont soumis. Ils travaillent essentiellement sur chant, et ont une tendance marquée à l'auto-forage sous les pressions dues à la mastication.

Ces implants, insérés en plan vertical, supportent mal les forces de traction, les forces d'enfoncement et les forces latérales dans les quatre directions.

La mise en place de cet implant est une opération délicate. Il est nécessaire de former une rainure qui corresponde parfaitement aux dimensions de la lame de LINKOW.

La mise en place d'un implant décrit dans le brevet Juillet, nécessite un fraisage qui ne peut actuellement que s'effectuer en deux temps d'intervention: un fraisage vertical puis un fraisage horizontal ou inversement, et ce avec des outils utilisés séparément. D'autre part, les instruments de fraisage de l'os sont soit en tungstène soit en acier, soit dans un autre matériau qui est toujours différent de celui de l'implant lui-même. Ceci comporte un risque de polymétallisme lorsqu'on place l'implant dans son site ou logement. En effet, cet implant étant en titane, les molécules de titane vont intérferer avec celles de tungstène ou d'acier ou du matériau autre que le titane.

Il est unanimement reconnu que lors du fraisage de l'os à l'aide d'une pièce métallique, il y a toujours des molécules de métal qui restent dans la région (territoire osseux). Il faut donc que l'outil et l'implant soient exécutés dans le même métal pour éviter le bimétallisme générateur de lésion tissulaire.

Deux autres brevets peuvent compléter l'état de la technique:

le brevet FR—A—1 551 020 SCIALOM. Ce brevet décrit un implant autoforant à forage vertical et à insertion verticale avec donc tous les inconvénients déjà décrits plus haut.

le brevet FR—A—2 302 715 CLUNET COSTE MANEUF décrit un implant dentaire. Cet implant est constitué de deux pièces à savoir un insert 1, de forme générale en T et d'un écrou 2 se vissant sur le précédent. L'insert est destiné à être introduit dans une tranchée en forme de T correspondante, ménagée dans un os maxillaire 3 et comportant une tranchée horizontale 4 au milieu de laquelle débouche une tranchée verticale 5. Suivant l'invention, l'insert 1 est constitué d'une seule pièce et il comporte une semelle 6 de laquelle part une tige perpendiculaire comprenant un fût 7. Ce fût présente, à sa partie supérieure, c'est-à-dire à l'opposé de la semelle 6 un filetage 8 sur lequel vient se visser l'écrou 2. Ce filetage 8 se termine par un cône 9 facilitant l'introduction de l'écrou.

L'invention tend à résoudre tous ces inconvénients. En particulier, l'invention tend à assurer une fixation primaire parfaite grâce à la précision avec laquelle le logement de l'implant est taillé dans l'os.

Implant dentaire autoforant pour la fixation de prothèses dentaires, ledit implant dentaire pouvant être en même temps son propre outil de mise en place, l'implant autoforant est constitué d'une partie qui fait office de fraise verticale à fraisage latéral et d'une ou de plusieurs parties qui font office de fraise roue horizontale, ledit implant autoforant réalisant ainsi, en même temps une micro-ostéotomie par fraisage latéral, simultanément dans les plans horizontal et vertical, et une insertion latérale de l'implant.

Cet implant autoforant possède une double fonction:

1) Il s'utilise comme une racine artificielle en remplacement des piliers dentaires naturels absents. C'est sa fonction principale et ultime.

2) Il peut être en même temps son propre outil de forage. Sa structure dentelée (sur la roue) et moletée sur l'axe, permet à cet implant, une fois monté sur un instrument rotatif (grâce à son embout lisse "de liaison"), d'être utilisé comme outil de fraisage de la loge implantaire (os maxillaire) destiné à le recevoir.

L'embout lisse faisant suite à la partie moletée et au filetage éventuel a été spécialement conçu pour pouvoir s'adapter directement sur une turbine ou un micro-moteur à grande vitesse. Eventuellement, une échancrure supplémentaire, a été aussi prévue sur l'embout de certains axes pour pouvoir recevoir la griffe de blocage des contre-angles à petite et moyenne vitesse et pièce à main.

La particularité de cet implant autoforant, c'est de réaliser en un seul temps une micro-ostéotomie par fraisage, simultanément dans les plans horizontal et vertical, autrement dit dans deux plans perpendiculaires.

Autre avantage de l'implant autoforant: cet impant fraise est animé (une fois clipsé sur la turbine, le micro-moteur ou le contre-angle ou la pièce à main) d'un mouvement de rotation autour de son axe.

En le plaçant latéralement contre la paroi osseuse, le praticien dans un mouvement rectiligne de translation, permet la pénétration intra et trans-osseuse dans un plan parallèle à celui de la roue dentelée, pour ce qui est de la base ou roue. Dans le même temps, il réalise une pénétration simultanée de la tige dans un plan perpendiculaire à la base ou roue.

Selon un autre mode de réalisation, l'implant autoforant peut être récupéré et un implant, correspondant au logement formé, peut être mis en place. Dans cette optique, l'implant qui doit être mis en place a des caractéristiques légèrement différentes par rapport à l'outil et ce, de manière à pouvoir être mis en place dans ledit logement "en force". Ainsi, le profil de l'implant est identique à celui de l'outil ou implant autoforant, mais il n'y a pas d'affûtage de certaines parties, d'autres parties sont plus épaisses pour éviter toute mobilité de l'implant dans son logement. La base de la roue a un rebord lisse ou lieu d'être dentelé. La tige peut être, soit lisse, soit filetée.

L'implant dentaire autoforant est conformé pour être disposé dans l'os mandibulaire ou maxillaire et fait ainsi office de racine artificielle.

Il peut être en même temps son propre outil de mise en place, comportant une tige dite "faux moignon" pour la fixation de prothèses dentaires fixes. L'extrémités supérieure de ladite tige est pourvue d'un moyen d'accrochage et d'une partie faisant office d'embout de liaison pour un moyen d'entraînement qui anime d'un mouvement de rotation ledit implant autour de son axe.

L'autre extrémité de la tige comporte une partie moletée faisant office de fraise verticale à fraisage latéral se terminant par une ou plusieurs parties perpendiculaires à l'axe longitudinal et fait ou font office de fraise roue, l'implant outil réalise ainsi, en même temps une micro-ostéotomie par fraisae latéral simultanément dans le plan horizontal et dans le plan vertical et en réalisant une insertion latérale de l'implant.

La partie moletée de la tige est formée de gorges ou rainures circulaires dont les arêtes sont affutées et forment des molettes rainurées dans le sens de l'axe longitudinal de la tige. Ledit rainurage dans le sens de l'axe de la tige est interrompu, à intervalles réguliers, par un crantage régulier.

Chaque roue comporte à sa périphérie des dents affutées.

La roue est amovible au niveau de la base de la tige.

L'implant dentaire autoforant comporte au moins deux roues dentelées perpendiculaires à l'axe longitudinal de la tige.

La partie moletée de la tige qui est située à la base de ladite tige, à partir de la roue dentelée, est formée de gorges ou rainures circulaires dont les arêtes sont affûtées. Cette partie peut donc couper verticalement lorsque la tige est entrânée par un instrument rotatif.

Le rainurage a été étudié de façon particulière pour obtenir une action sécante maximale pour un minimum d'échauffement et de traumatisme au niveau du tissu osseux. Il y a aussi un crantage dans le sens horizontal qui permet d'évacuer les copeaux, tout en diminuant l'échauffement du tissu osseux.

La roue dentelée comporte, à sa périphérie, des dents affûtées. Elle peut couper horizontalement lorsque ladite tige est entraînée par un instrument rotatif.

La roue dentelée peut comporter des trous qui permettent d'alléger le poids de la matière de l'implant-outil, et ce, sans diminuer les qualités mécaniques.

Le procédé d'insertion d'un tel implant autoforant consiste à emmancher la turbine sur l'embout de liaison dudit implant-outil, dès qu'il y a rotation; on peut réaliser, en un seul temps, une micro-ostéosection par fraisage, simultanément dans un plan horizontal et dans un plan vertical, soit dans deux plans perpendiculaires. Le nombre de roues détermine le nombre de plans horizontaux.

L'implant-autoforant est mis en place dans le logement formé par la découpe correspondant au profil projeté latéralement de l'outil ou de l'implant-outil. L'implant autoforant ou l'implant indépendant est inséré latéralement dans ladite découpe, de préférence par la face vestibulaire, de manière que seule dépasse de la crête de l'os l'extrémité de la tige opposée à celle de la roue. La tige est coupée à la hauteur voulue pour recevoir la vis ou bague taraudée. Il n'y a pas d'ajustage puisque l'outil et l'implant ne forment qu'une seule pièce. Avant son blocage biologique définitif par régénération de l'os autour de l'implant et notamment au niveau de la partie moletée, ledit implant est parfaitement stable de par la qualité des caractéristiques techniques de son logement.

Selon un autre mode de réalisation, l'implant dentaire peut ne pas être autoforant et ne pas être ainsi son propre outil.

L'implant dentaire pour la fixation de prothèses dentaires fixes est destiné à être inséré latéralement dans un logement par un fraisage latéral par l'implant autoforant. Ledit implant est constitué d'une semelle conformée pour être disposée dans l'os mandibulaire ou maxillaire. La semelle conformée est surmontée d'une tige dite "faux moignon" pour la fixation de prothèses dentaires fixes. L'extrémité supérieure de la tige est pourvue d'un moyen d'accrochage qui permet de fixer une bague taraudée ou vis sur laquelle est mis en place la prothése dentaire. La semelle est formée d'au moins une ou plusieurs roues perpendiculaires à l'axe longitudinal de la tige. Les roues sont lisses à leur périphérie avec une action rétentive et sont légèrement supérieure en épaisseur à l'épaisseur de la ou des roues correspondantes utilisées par l'outil, de manière à pouvoir pénétrer en force dans le logement créé par l'outil.

La tige a une partie molétée s'étendant sur une certaine hauteur au-dessus des roues, et comporte sur sa périphérie des molettes longitudinales lisses dont les arêtes sont moins vives que celles de l'implant autoforant. La partie moletée est entrecoupée régulièrement sur sa hauteur par des gorges ou rainures circulaires dont les arêtes sont moins vives que celles de l'implant autoforant, ayant ainsi un profil conformé pour réaliser une action rétentive.

Les dessins ci-joints donnés à titre d'exemple indicatif et non limitatif permettront aisément de comprendre l'invention. Ils représentent des modes de réalisation préférés selon l'invention.

La figure 1 est une vue de côté de l'outil ou de l'implant-outil.

La figure 2 est une vue en coupe de l'outil ou de l'implant-outil selon l'axe B—B représenté dans la figure 1.

La figure 3 est une vue de côté de l'implant indépendant de l'outil.

La figure 4 est une vue en coupe selon l'axe A—A de l'implant indépendant de l'outil, tel que représenté à la figure 3.

La figure 5 est une vue schématique d'un outil en rotation en cours de travail pour découper dans l'os maxillaire un logement implantaire.

La figure 6 est une vue schématique d'un outil qui, par sa rotation selon la flèche F1, a creusé le logement ou le site pour l'implant dans l'os maxillaire.

La figure 7 met en évidence que l'implant-outil, ou l'outil, peut entrer et sortir latéralement du logement de l'impant selon les flèches F2, F3. Il suffit d'insérer latéralement l'implant dans la découpe créée par le profil de l'implant-outil ou de l'outil.

La figure 8 représente un implant indépendant qui va être introduit en force selon la flèche F4 dans son logement. Il y a lieu de noter que les gorges ou rainures ne sont pas affûtées, que l'épaisseur de la roue lisse est plus grande que l'épaisseur de la roue dentelée de l'outil, ce qui assure une mobilité parfaite de l'implant dans son logement ou site.

La figure 9 est une vue schématique d'un implant indépendant de l'outil, mis en place dans son logement implantaire, l'extrémité de l'implant comporte un moyen d'attache, tel par exemple un filetage qui permet de venir fixer le fût, sur cette bague taraudée ou vis sera cimentée la prothèse dentaire.

La figure 10 est une vue en perspective d'une mâchoire inférieure mettant en évidence des implants en place, avec leur bague taraudée ou vis prête à recevoir la prothèse, un outil en cours de travail, un logement ou site pour implant.

Les figurse 11, 12, 13 mettent en évidence le travail de l'implant-outil qui creuse dans l'os une découpe qui correspond à son profil.

La figure 14 met en évidence comment l'embout de liaison permet de fixer l'outil ou l'implant-outil dans le mandrin de la turbine.

La figure 15 met en évidence la turbine en action avec des jets d'air et d'eau pour le refroidissement.

L'implant autoforant 1 selon l'invention est constitué d'une partie 2 qui fait office de fraise verticale et d'une autre partie 3 perpendiculaire à l'axe longitudinal et qui fait office de fraise roue 5. La partie 2 comporte à sa base 4 une roue 5 dentelée perpendiculaire à l'axe longitudinal et à son autre extrémité 7 un moyen d'accrochage 6 qui permet de fixer la bague taraudée ou vis 8 sur laquelle est mise en place la prothèse dentaire 9 et/ou une partie lisse qui fait office d'embout de liaison 11.

L'implant autoforant 1 est fait d'une seule pièce, constituée par une tige 10 présentant un embout de liaison 11 qui s'emmanche dans le mandrin de la turbine 23, cet embout de liaison 11 peut comporter à son extrémité 7, un moyen de fixation 6 qui permet la mise en place d'une bague taraudée ou vis 8. Ledit moyen de fixation 6 peut être par exemple un filetage de cette extrémité 7 de l'implant-outil 1. Le profil de la bague taraudée ou vis est particulier. Il est formé de deux troncs de cône inversés, a et b, un grand et un petit.

"a" est le grand tronc de cône: il est destiné à recevoir la dent ou la supra structure prothétique.

"b" est le petit tronc de cône: il jouxte la gencive et a une forme qui permet au tissu gingival de venir épouser son contour de façon parfaite. Ceci limite les risques d'irritation et de rétention de la plaque bactériènne et des débris alimentaires. Le tronc de cône inversé b suprime tout surplomb et par la même le blocage des matières à ce niveau. Cette forme tient compte des concepts parodontaux les plus actuels.

L'autre extrémité 2 de l'implant-outil 1 comporte une partie moletée 12 qui se termine par une roue dentelée 5 perpendiculaire à l'axe longitudinal.

La partie moletée 12 est formée de gorges ou rainures circulaires 13 dont les arêtes sont affûtées et forment des molettes 22 rainurées dans le sens de l'axe longitudinal de la tige, de manière à obtenir une action sécante maximale pour un minimum d'échauffement et de traumatisme osseux. Il exite également un crantage dans le sens horizontal qui permet d'évacuer les copeaux tout en diminuant l'échauffement du tissu osseux.

La roue 5 comporte à sa périphérie des dents affûtées 14.

La roue 5 peut comporter, par exemple, quatre trous 15 qui permettent d'alléger le poids de matière de l'implant-outil et ce, sans diminuer les qualités mécaniques. C'est par ces orifices que le tissu osseux pénètre au travers de l'implant et assure une partie du blocage biologique définitif. Le reste du blocage étant assuré par l'emprisonnement dans l'os de l'ensemble base plus tige.

Selon un autre mode de réalisation, l'implant dentaire n'est pas autoforant et ne peut constituer son propre outil.

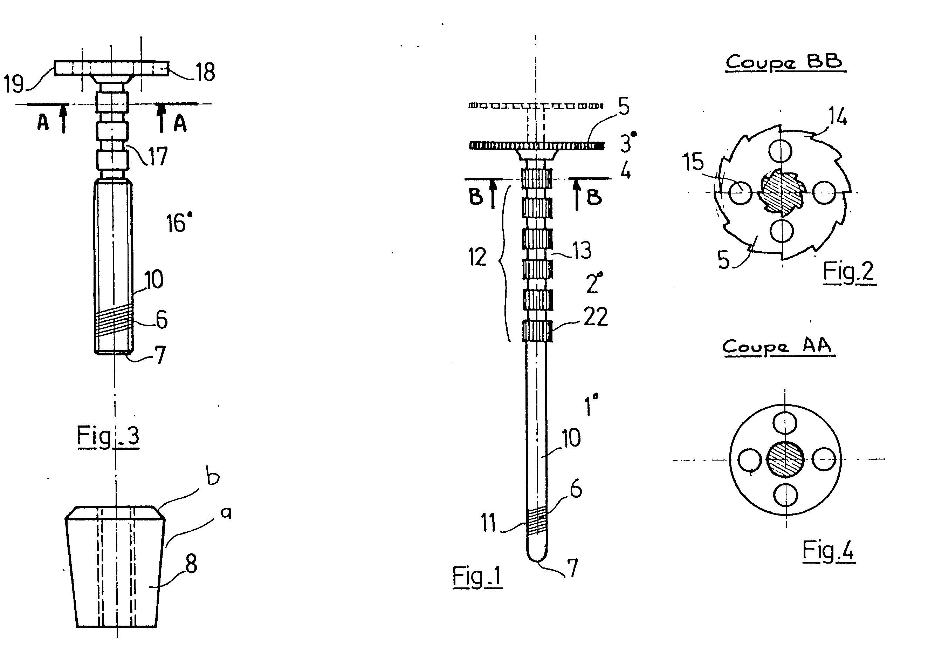

1) L'implant autoforant comporte toutes les caractéristiques techniques ci-dessus décrites pour l'implant-outil (voir la figure 1).

2) L'implant proprement dit 16 comporte toutes les caractéristiques techniques ci-dessus décrites pour l'implant-outil 1 avec les différences techniques essentielles suivantes (voir la figure 3):

—il est constitué d'une tige 10 comportant des molettes 22 lisses dans le sens de l'axe longitudinal de la tige et des gorges ou rainures circulaires 17 dont les profils ont uniquement une action rétentive et d'une ou plusieurs roues horizontales 18 perpendiculaires à l'axe de ladite tige 10 et dont la périphérie est lisse.

—les proportions dudit implant 16 sont légèrement plus grandes que celles du logement prévu à son insertion latérale afin de permettre une pénétration en force.

—la partie moletée 12 n'a plus une action sécante verticale, donc les crêtes des gorges ou rainures 17 sont moins vives de même pour les molettes 22. Cette partie n'a plus d'action sécante, mais une action rétentive. Les gorges circulaires 17 sont donc moins affûtées, les arêtes sont donc moins vives,

—la roue lisse 18, perpendiculaire à l'axe de la tige ne comporte pas de dents à sa périphérie, de plus, son épaisseur 19 est plus grande que l'épaisseur de la roue dentelée 5 de l'outil 1 ou implant-outil et ce de manière à pouvoir pénétrer "en force" dans le logement implantaire 20 créé par la roue dentelée 5 de l'outil 1.

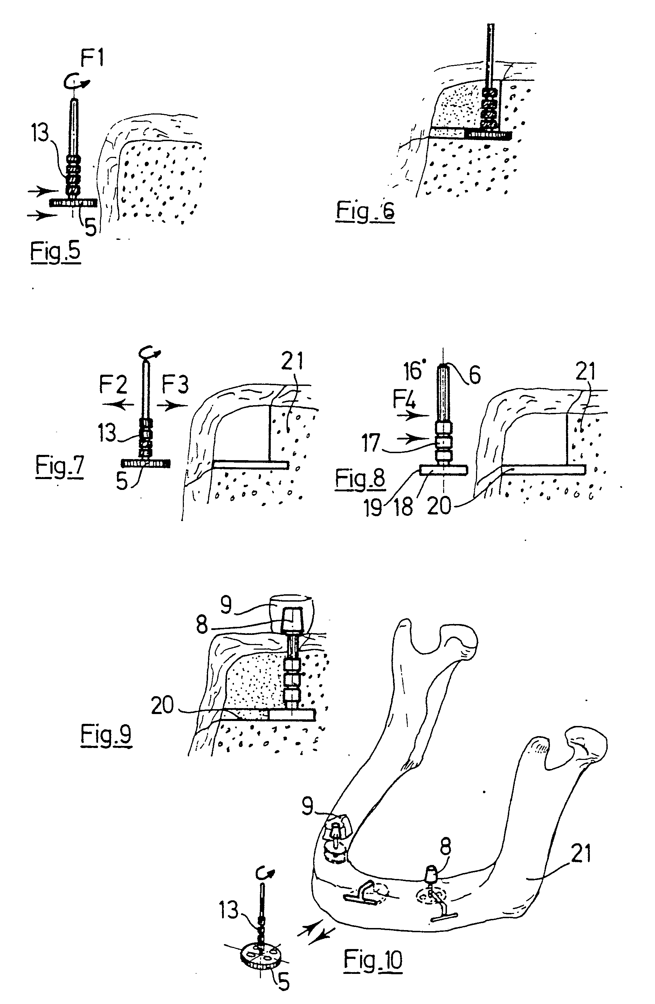

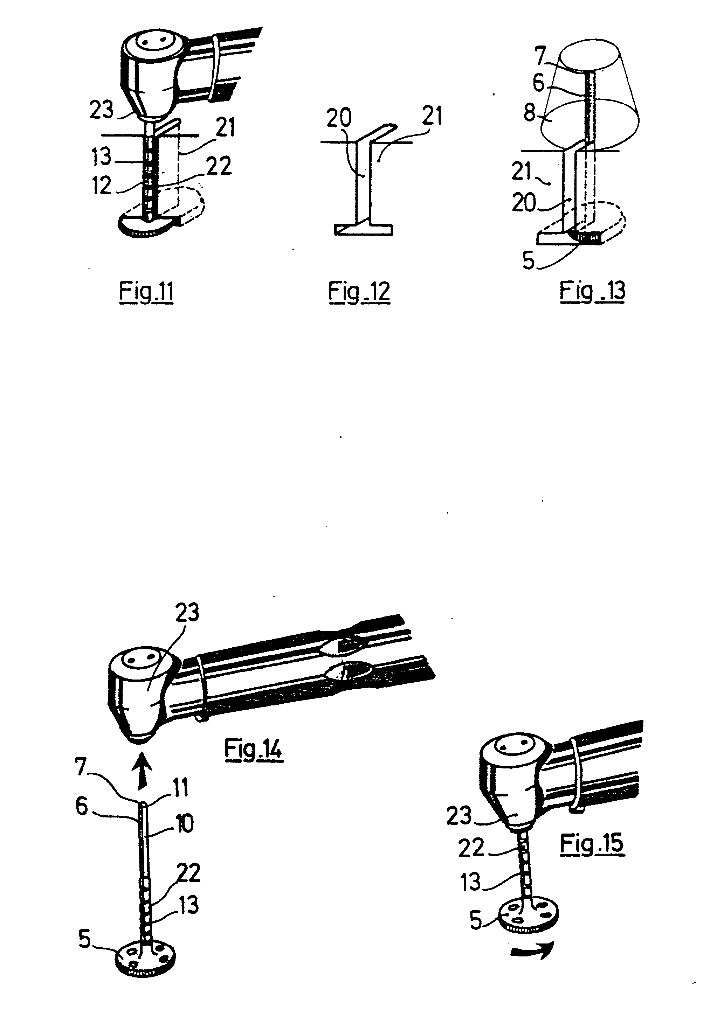

La figure 11 met en évidence l'action de taille, de l'outil ou de l'implant-outil dans l'os pour pratiquer une découpe correspondant au logement ou site implantaire 20. Ce logement 20 correspond au profil projeté latéralement dudit outil ou implant-outil à insérer latéralement dans ladite découpe. Seule dépasse de la crête de l'os 21, l'extrémité 7 de la tige 10 opposée à la roue dentelée 5 ou à la roue lisse 18 perpendiculaire à l'axe longitudinal de ladite tige 10. La figure 9 met en évidence l'implant 1 ou l'implant-outil 16 en place, muni de la dent prothétique 9 après que la tige 10 ait été coupée à la hauteur voulue et qu'elle ait reçu la vis filetée 8 correspondante.

## Revendications

1. Implant dentaire autoforant (1) conformé pour être disposé dans l'os mandibulaire ou maxillaire et faisant ainsi office de racine artificielle, ledit implant dentaire pouvant être en même temps son propre outil de mise en place, comportant une tige (10) dite "faux moignon" pour la fixation de prothèses dentaires fixes (9), l'extrémité supérieure (7) de ladite tige (10) étant pourvue d'un moyen d'accrochage (6) sur lequel est mis en place la prothèse dentaire et une partie qui fait office d'embout de liaison (11) pour un moyen d'entraînement qui anime d'un mouvement de rotation ledit implant autour de son axe, caractérisé par le fait que l'autre extrémité (2) de la tige (10) comporte une partie moletée (12) qui fait office de fraise verticale à fraisage latéral qui se termine par une ou plusieurs parties (3) perpendiculaires à l'axe longitudinal et qui fait ou font office de fraise roue (5), ledit implant outil (1) rélisant ainsi, en même temps une micro-ostéotomie par fraisage latéral, simultanément, dans le plan horizontal et dans le plan vertical et une insertion latérale de l'implant.

2. Implant dentaire autoforant selon la revendication 1, caractérisé par le fait que la partie moletée (12) de la tige (10) est formée de gorges ou rainures circulaires (13) dont les arêtes sont affutées et forment des molettes (22) rainurées dans le sens de l'axe longitudinal de la tige (10), ledit rainurage dans le sens de l'axe de la tige (10) est interrompu, à intervalles réguliers, par un crantage régulier.

3. Implant dentaire autoforant selon la revendication 1, caractérisé par le fait que chaque roue (5) comporte à sa périphérie des dents affutées.

4. Implant dentaire autoforant selon la revendication 1, caractérisé par le fait que la roue (5) est amovible au niveau de la base (4) de la tige (10).

5. Implant dentaire autoforant selon la revendication 1, caractérisé par le fait qu'il comporte au moins deux roues dentelées (5) perpendiculaires à l'axe longitudinal de la tige (10).

6. Implant dentaire (16) pour la fixation de prothèses dentaires fixes destiné à être inséré latéralement dans un logement formé par un fraisage latéral par l'implant autoforant tel que défini dans les revendications précédentes, ledit implant étant constitué d'une semelle conformée pour être disposée dans l'os mandibulaire ou maxillaire, ladite semelle étant surmontée d'une tige (10) dite "faux moignon" pour la fixation de prothèses dentaires fixes (9), l'extrémité supérieure (7) de ladite tige (10) est pourvue d'un moyen d'accrochage (6) qui permet de fixer une bague taraudée ou vis (8) sur laquelle est mis en place la prothèse dentaire (9), caractérisé par le fait que ladite semelle soit formée d'au moins une ou plusieurs roues (18) perpendiculaires à l'axe longitudinal de ladite tige, lesdites roues étant lisses à leur périphérie avec une action rétentive et étant légèrement supérieure en épaisseur (19) à l'épaisseur de la ou des roues correspondantes utilisées par l'outil, de manière à pouvoir pénétrer en force dans le logement créé par l'outil, la tige ayant une partie moletée (12) s'étendant sur une certaine hauteur au-dessus desdites roues, et comportant sur sa périphérie des molettes longitudinales (22) lisses dont les arêtes sont moins vives que celles de l'implant autoforant, ladite partie moletée étant entrecoupée régulièrement sur sa hauteur par des gorges ou rainures circulaires (17) dont les arêtes sont moins vives que celles de l'implant autoforant, ayant ainsi un profil conformé pour réaliser une action rétentive.

**Patentansprüche**

1. Selbstbohrendes Zahnimplantat (1) zum Einsetzen entweder in den Unterkiefer- oder in den Oberkieferknochen unter Bildung einer künstlichen Wurzel, wobei das Zahnimplantat gleichzeitig sein eigenes Einsetzwerkzeug sein kann und einen Schaft (10), genannt "falscher Stumpf", zur Anbringung fester Zahnprothesen (9) aufweist, wobei das obere Ende (7) des genannten Schaftes (10) mit einer Verankerung (6) versehen ist, auf welcher die Zahnprotese angeordnet ist, sowie ein Teil, das als Verbindungsstück (11) für eine Antriebseinrichtung dient, welche das Implantat umn seine Achse dreht, dadurch gekennzeichnet, daß das andere Ende (2) des Schaftes (10) einen gerieften Teil (12) aufweist, welcher als Senkrechtfräser mit seitlicher Fräsung dient und in einem oder mehreren Teilen (3) senkrecht zur Längsachse endet, welcher beziehungsweise welche als Rundlauffräse (5) dient/dienen, wodurch das genannte Implantat-Werkzeuge (1) gleichzeitig sowohl in der horizontalen als auch in der vertikalen Ebene durch seitliche Fräsung eine Mikroosteotomie als auch ein seitliches Einsetzen des Implantats ermöglicht.

2. Selbstbohrendes Zahnimplantat nach Anspruch 1, dadurch gekennzeichnet, daß der geriefte Teil (12) des Schaftes (10) kreisförmige Ausnehmungen oder Nuten (13) aufweist, deren Kanten zugeschliffen sind und in Richtung der Längsachse des Schaftes (10) geriefte Rädchen (22) bilden, wobei die genannte Riefung in Achsrichtung des Schaftes (10) in regelmäßigen Abständgen durch eine Rastung unterbrochen ist.

3. Selbstbohrendes Zahnimplantat nach Anspruch 1, dadurch gekennzeichnet, daß jedes Rädchen (5) an seinem Umfang zugeschliffene Zähne aufweist.

4. Selbstbohrendes Zahnimplantat nach Anspruch 1, dadurch gekennzeichnet, daß das Rädchen (5) in Höhe der Basis (4) der Schafts (10) unbeweglich ist.

5. Selbstbohrendes Zahnimplantat nach Anspruch 1, dadurch gekennzeichnet, daß dieses mindestens zwei, Sägezähne aufweisende, Rädchen (5) senkrecht zur Längsachse des Schaftes (10) aufweist.

6. Zahnimplantat (16) zur Anbringung fester Zahnprothesen zur seitlichen Einbringung in eine mittels Fräsung durch das selbsbohrende Implantat gebildete Aufnahme gemäß den vorangehenden Ansprüchen, wobei das Implantat ein entsprechend ausgebildetes Fundament für die Einbringung in den Oberkiefer- beziehungsweise Unterkieferknochen bildet, wobei das genannte Fundament einen Schaft (10), genannt "falscher Stumpf", zur Anbringung fester Zahnprothesen (9) trägt und das obere Ende (7) des genannten Schaftes (10) mit einer Verankerung (6) zur Anbringung eines Ringes mit Gewindebohrung oder einer Schraube (8) versehen ist, auf welchem/welcher die Zahnprotese (9) angeordnet ist, dadurch gekennzeichnet, daß das genannte Fundament aus mindestens einem oder aber mehreren Rädchen (18) senkrecht zur Längsachse des genannten Schaftes gebildet ist, wobei die genannten Rädchen an ihrem Umfang mit rückhaltender Wirkung glatt ausgebildet sind und ihre Dicke (19) etwas größer ist als die des/der entsprechenden für das Werkzeug verwendeten Rädchen(s), dergestalt, daß es/sie unter Ausübung von Kraft in die von dem Werkzeug geschaffene Aufnahme hineindrückbare ist/sind, wobei der Schaft einen gerieften Teil (12) aufweist, welcher sich über eine bestimmte Höhe über den genannten Rädchen erstreckt und an seinem Umfang glatte geriefte Längsrädchen (22) aufweist, deren Kanten weniger scharf sind als die des selbstbohrenden Implantats, wobei der geriefte Teil regelmäßig über seine Höhe von kreisförmigen Ausnehmungen beziehungsweise Nuten (17) unterbrochen ist, deren Kanten weniger scharf zugeschliffen sind als die des selbstbohrenden Implantats, wodurch sie ein entsprechendes Profil mit rückhaltender Wirkung aufweisen.

**Claims**

1. Self-drilling dental implant (1) for insertion either in the mandible or the maxilla bones, serving as an artificial root, the dental implant being so designed as to, at the same time, constitute its own insertion tool and comprising a shaft (10), called a "false stump", for mounting fixed dental protheses (9), with the upper end (7) of the shaft (10) mentioned being provided with a stay (6) on which the dental prothesis is mounted, and comprising a part serving as connecting piece (11) for a driving means for rotating the implant about its axis, characterized in that the other end (2) of the shaft (10) comprises a fluted portion (12) serving as a vertical milling cutter active also in the lateral direction and ending in one or several portion(s) (3) vertically of the longitudinal axis, serving as a rotary milling cutter (5), whereby it is possible with said implant tool (1) to effect a micro-osteotomy simultaneously in the horizontal plane and in the vertical plane through cutting in the lateral direction, as well as a lateral insertion of the implant.

2. The self-drilling dental implant according to claim 1, characterized in that the fluted part (12) of the shaft (10) comprises circular recesses or grooves (13) whose edges are sharpened and define wheels (22) fluted in the direction of the longitudinal axis of the shaft (10), with said fluting, in the direction of the axis of the shaft (10), being intersected by reduced portions at regular intervals.

3. The self-drilling tooth implant according to claim 1, characterized in that each wheel (5) has sharpened teeth at its periphery.

4. The self-drilling tooth implant according to claim 1, characterized in that at the level of the basis (4) of the shaft (10) the wheel is immobile.

5. The self-drilling tooth implant according to claim 1, characterized in that perpendicularly of the longitudinal axis of the shaft (10) it comprises at least two wheels (5) having saw teeth.

6. Dental implant (16) for the mounting of fixed dental protheses intended to be inserted laterally in a recess formed by milling with the aid of the self-drilling implant as defined in the preceding claims, said implant constituting a correspondingly formed base apt to be inserted in the mandible or the maxilla bones, said base carrying a shaft (10), called "false stump", for the mounting of fixed dental protheses (9), and the upper end (7) of the shaft (10) being provided with a stay (6) permitting the fixing of a threaded ring or screw (8) on which the dental prothesis (9) is disposed, characterized in that said base is formed from at least one, if not several, wheels (18) perpendicular to the longitudinal axis of said shaft, with the wheels being smooth with a retentive effect at their peripheries, and their thickness (19) being slighly greater than that of the corresponding wheel(s) used for the tool so as to permit entry thereof under force into the recess provided by the tool, the shaft having a fluted portion (12) extending over a certian height above said wheels and comprising smooth fluted longitudinal wheels (22) at its periphery whose edges are less sharp than those of the self-drilling implant, with the fluted part being regularly intersected over its height by circular recesses or grooves (17) whose edges are less sharp than those of the self drilling implant, thus having a profile apt to provide a retentive effect.

19 18

A A

17

16°

10

6

7

Fig. 3

b

a

8

5

3°

4

B B

12

13

2°

22

1°

10

6

11

7

Fig. 1

Coupe BB

14

15

5

Fig. 2

Coupe AA

Fig. 4

EP 0 214 962 B1

EP 0 214 962 B1

Fig.5

F1

13

5

Fig.6

Fig.7

F2 F3

13

5

21

Fig.8

16 6

F4

17

19 18 20

21

Fig.9

8 9

20

9

8

21

13

5

Fig.10

2

Fig.11

Fig.12

Fig.13

Fig.14

Fig.15